# EUROPEAN PATENT APPLICATION

(11) **EP 2 494 868 A1**
(43) Date of publication of application: **05.09.2012**
(21) Application number: 09850765.0
(22) Date of filing: 26.10.2009
(51) Int. Cl.: A01N 65/00, A61K 8/97

(54) **METHOD FOR THE EXTRACTION AND HYDROLYSIS OF ANY PROTEIN SUBSTANCE, NATURAL AUXINS AND POLYPHENOLS FROM SOURCES OF PLANT ORIGIN, AND RESULTING PRODUCT**

(71) Applicant: Ciencia Intra-Vegetal, S.L., 46117 Betera - Valencia (ES)
(72) Inventor: PONS RAGA, Alberto, 46117 Betera - Valencia (ES)
(74) Representative: Urizar Anasagasti, Jesus Maria
(86) International application number: PCT/ES2009/070460
(87) International publication number: WO 2011/051508

(57) **Abstract**

Method for the extraction and hydrolysis of any protein substance, natural auxins and polyphenols from sources of plant origin and their derivatives, which from a any vegetable matter (roots, stems including bark, leaves, fruits, seeds and derivatives) and by an hydrolysis in acid alcohol which is added alcohols and mineral acids, the resulting mass is subjected to a thermodynamic treatment, with subsequent utilization of physical systems of separate solid /liquid plant extract is obtained a hydro alcoholic.

## Description

### OBJECT OF THE INVENTION

The object of this patent is to present a new method for the extraction and hydrolysis of any protein substance (especially defensins, thionins and rip's (the latter inhibitory proteins called ribosomes), natural auxin, and polyphenols from any source of plant origin (roots, stems including bark, leaves, fruits, seeds and derivatives), and product thus obtained.

With this new procedure yields a product which contains among other components peptides resulting from the alcoholic acidic hydrolysis of a small molecular weight. Many of these peptides are substances commonly called defensins, thionins'S AND RIP (ribosome-inhibiting proteins). In turn, polyphenols and auxins are released from their associations generating organic substances free in solution.

The application of these products as supplements in farming, can give plants an acquired immune system that is vital in the defense of plants against diseases caused by these supported, and also stabilizes the metabolic processes and adapt to situations stress or overexertion. Similarly applied, as an external, cosmetic uses to create defenses against infections, antioxidant systems and protective color of all external parts of mammals.

### BACKGROUND OF THE INVENTION

Currently there are several plant extracts on the market, which include many products that are derived from a plant source. Each of these products has a process to obtain proper to it, and that fits the general principle of obtaining the highest concentration of substances in these terms.

There alcoholic extracts, products of acid or alkaline hydrolysis, saline extracts, solvent extracts, but not described in the prior art method for obtaining a plant extract obtained by hydrolysis any acidic alcoholic whose purpose is to fractionate vegetable proteins into peptides (especially defensins, thionins and RIP's), and generate free molecules of the family of plant polyphenols and natural plant auxins.

### DESCRIPTION OF THE INVENTION AND PREFERRED EMBODIMENT

As an alternative to the procedures presented this new procedure for extraction and hydrolysis of any plant protein substance (especially defensins, thionins and RIP's), natural plant auxins, plant polyphenols from plant sources, and product thus obtained, the subject of this patent.

In this procedure starting from a vegetable material either (roots, stems including bark, leaves, fruits, seeds and derivatives), it proceeds to an acidic alcoholic hydrolysis consisting of the addition of linear alcohols within the range from 1 carbon atom in the molecule up to 8 carbon atoms, ie from the methanol to octanol, together with mineral acids. In amounts ranging from 10% to 60% of the final mass obtained in the addition of alcohols, and 1% to 30% of the final mass obtained in the addition of any mineral acid, the addition of these ingredients, generates the optimal mix of which is necessary to extract and hydrolyzed vegetable protein any substance (especially defensins, thionins and RIP's), natural plant auxins and plant polyphenols. Depending on the source and wealth of plant material used will result in a more or less liquid wealth of target substances.

The resulting mixture is subjected to conditions established thermodynamic temperature control from 50 ° C to 125 ° C and pressure in the range 0.1 to 1.1 atmospheres atmospheres of pressure. The reaction is a simple process reflux with absolute control of temperature and pressure. Each fraction obtained peptide requires a temperature optimum of production. The diverse nature of the peptides (especially defensins, thionins and RIP's), polyphenols and natural auxins wanted us to perform temperature scaling for all these substances are produced.

Then proceed to the use of physical systems of solid / liquid separation either, as the concentration systems based on molecular exclusion techniques, nanofiltration, or liquid chromatography; obtaining vegetable extracts concentrated and fractionated according to molecular size of its component peptides (especially defensins, thionins and RIP's) natural auxins and polyphenols.

The percentage of peptides (especially defensins, thionins and RIP's), natural auxins and polyphenols will depend on the plant material in the splitting.

Having sufficiently described the nature of the present invention, as well as a way of putting it into practice, only be added that the invention may undergo certain variations in the procedure and composition, provided that such alterations do not vary substantially the features that are claimed below.

## Claims

1. Method for the extraction and hydrolysis of any protein substance, natural auxins and polyphenols from plant sources and product thus obtained, wherein material from a plant either proceed to the completion of alcoholic acid hydrolysis.

2. Method for the extraction and hydrolysis of any protein substance, natural auxins and polyphenols from plant sources and the product obtained according to the first claim, **characterized in that** the alcoholic acidic hydrolysis involves the addition of linear alcohols within the range ranges from 1 carbon atom in the molecule up to 8 carbon atoms, ie from the methanol to octanol, together with mineral acids in amounts ranging from 10% to 60% of the final mass obtained in the addition of alcohols, and 1% to 30% of the final mass obtained in the addition of any mineral acid, the addition of these ingredients, generates the optimum mixture which is necessary to extract and hydrolyze all protein substances, natural auxins and polyphenols plant, according to the origin and wealth of plant material used, will result in a more or less liquid wealth in the structure of target substances.

3. Method for the extraction and hydrolysis of any protein substance, natural auxins and polyphenols from plant sources and product obtained according to claim first and second, wherein the resulting mixture is subjected to a thermodynamic conditions which sets controlling temperatures from 50 ° C to 105 ° C and pressure in the interval 0.1 atmospheres to 1.1 atmospheres of pressure, even so, the kinetics of the reaction requires strict control of temperature - pressure - time to get the diverse group of peptides plants, auxins natural plant and plant polyphenols sought and the wide range of temperatures is due to the diverse nature of plant peptides, natural plant auxins and plant polyphenols that wanted us to do a scaled temperature for the products sought to be produced.

4. Method for the extraction and hydrolysis of any protein substance, natural auxins and polyphenols from plant sources and the product obtained according to the preceding claims, **characterized by** the use of physical systems of solid / liquid separation either, and the concentration systems based on molecular exclusion techniques, nanofiltration, or liquid chromatography; obtaining vegetable extracts concentrated and fractionated according to molecular size of its component peptides, natural auxins and polyphenols.

5. Method for the extraction and hydrolysis of any protein substance, natural auxins and polyphenols from plant sources and resulting product wherein the product obtained is hydro alcoholic plant extracts with a peptide content of a size from 1 kDa up to 100 kDa from 1% to 50%.

6. Method for the extraction and hydrolysis of any protein substance, natural auxins and polyphenols from plant sources and the product obtained according to the fifth claim, wherein the product obtained is hydro alcoholic plant extracts having a content of natural plant auxin from 0.1% to 15% and plant polyphenols from 0.1% to 15%.
